# EUROPEAN PATENT SPECIFICATION

(11) **EP 4 140 429 B1**
(45) Date of publication and mention of the grant of the patent: **17.12.2025**
(21) Application number: 22191505.1
(22) Date of filing: 22.08.2022
(51) Int. Cl.: A61B 18/14, A61B 18/00, A61B 18/12

(54) **BASKET CATHETER WITH POROUS SHEATH**
KORBKATHETER MIT PORÖSER HÜLLE
CATHÉTER À PANIER À GAINE POREUSE

(30) Priority: 23.08.2021 US 202163236135 P; 01.11.2021 US 202163274334 P; 12.08.2022 US 202217887201
(43) Date of publication of application: 01.03.2023
(73) Proprietor: BIOSENSE WEBSTER (ISRAEL) LTD., 2066717 Yokneam (IL)
(72) Inventor: GOVARI, Assaf, Yokneam 2066717 (IL); ALTMANN, Andres Claudio, Yokneam 2066717 (IL); BEECKLER, Christopher Thomas, Irvine 92618 (US); KEYES, Joseph Thomas, Irvine 92618 (US); LICHTER, Justin George, Irvine 92618 (US)
(74) Representative: Carpmaels & Ransford LLP

(56) References cited:
- EP-A1- 3 153 124
- US-A1- 2003 236 455
- US-A1- 2013 096 550
- US-A1- 2017 311 829

## Description

### FIELD OF THE DISCLOSURE

The present disclosure relates generally to invasive medical equipment, and particularly to apparatus for ablating tissue within the body and methods for producing and using such apparatus. The invention provides a medical apparatus according to claim 1 and a method for producing a medical device according to claim 13. The disclosed methods of use do not form part of the invention, but are provided for better understanding of the invention.

### BACKGROUND

Cardiac arrythmias are commonly treated by ablation of myocardial tissue in order to block arrhythmogenic electrical pathways. For this purpose, a catheter is inserted through the patient's vascular system into a chamber of the heart, and an electrode or electrodes at the distal end of the catheter are brought into contact with the tissue that is to be ablated. In some cases, high-power radio-frequency (RF) electrical energy is applied to the electrodes in order to ablate the tissue thermally. Alternatively, high-voltage pulses may be applied to the electrodes in order to ablate the tissue by irreversible electroporation (IRE).

Some ablation procedures use basket catheters, in which multiple electrodes are arrayed along the spines of an expandable assembly at the distal end of the catheter. The spines bend outward to form a basket-like shape and contact tissue within a body cavity. For example, U.S. Patent Application Publication 2020/0289197 describes devices and methods for electroporation ablation therapy, with the device including a set of spines coupled to a catheter for medical ablation therapy. Each spine of the set of splines may include a set of electrodes formed on that spine. The set of spines may be configured for translation to transition between a first configuration and a second configuration.

US 2013/096550 A1 discloses medical devices and methods for making and using medical devices. EP 3 153 124 A1 discloses a catheter adapted for pulmonary vein isolation, has a distal electrode assembly that can sit stably in an ostium as secured by a plurality of spines and a membrane member. US 2003/236455 A1 discloses a probe assembly for mapping and ablating pulmonary vein tissue and method of using the same. US 2017/311829 A1 discloses a method of constructing an electrophysiology catheter having a flex circuit electrode assembly includes: providing a flex circuit having a substrate, a first conductive layer and a second conductive layer; removing the first conductive layer to expose a first surface of the substrate; forming the wiring electrode in the second conductive layer with one exclusion zone; forming a first through-hole in the substrate to provide one conductive via and forming a second through-hole to provide an irrigation aperture in alignment with the exclusion zone; forming the contact electrode on first surface of the substrate; placing conductive material into the first through-hole to form the conductive via, the conductive via extending through the substrate and electrically coupling the wiring electrode and the contact electrode; and coupling a first conductor and a second conductor to the wiring electrode to form a thermocouple.

The present disclosure will be more fully understood from the following detailed description of the examples thereof, taken together with the drawings in which:

### BRIEF DESCRIPTION OF THE DRAWINGS

Fig. 1 is a schematic pictorial illustration showing a system for cardiac ablation, in accordance with an embodiment of the invention;
Fig. 2 is a schematic side view of a catheter expandable assembly with a porous sheath, in accordance with an embodiment of the invention;
Figs. 3A and 3B are schematic cutaway views of the catheter expandable assembly of Fig. 2 in collapsed and expanded configurations, respectively, in accordance with an embodiment of the invention;
Fig. 4 is a flow chart that schematically illustrates a method for producing a sheath for a catheter expandable assembly, in accordance with an embodiment of the invention;
Fig. 5 is a schematic side view of a system for producing sheaths for catheter basket assemblies, in accordance with an embodiment of the invention;
Fig. 6 is a schematic side view of a braided tube produced using the system of Fig. 5, in accordance with an embodiment of the invention;
Fig. 7A is a side view of an exemplary expandable member; and
Fig. 7B is a side view of yet another expandable member that can be used with the sheaths produced from the braided tube of Fig. 6.

### DETAILED DESCRIPTION OF EXAMPLES

Catheters with expandable assemblies, such as basket catheters, are useful in performing ablation procedures rapidly and efficiently, since the spines of the basket catheter (and thus the electrodes on the spines) are able to contact and ablate the tissue at multiple locations concurrently. The spines themselves, however, can give rise to dangerous blood clots during the ablation procedure, due to the disturbance they cause in the blood flow, as well as due to arcing between the spines, particularly in IRE-based ablation. Furthermore, a spine can become embedded in the tissue during the procedure, which can lead to local overheating, resulting in charring and/or other trauma. The use of spines having smooth, rounded profiles can be helpful in mitigating these effects, but by itself does not eliminate the problems of clotting and tissue damage.

Examples of the present disclosure that are described herein address these problems by covering the expandable assembly with a porous sheath. (As used herein, the term "sheath" is intended to include "an outer cover" or a "membrane.") The sheath prevents direct contact between the spines and the tissue, while still permitting electrical energy to be applied from the electrodes through the sheath to the tissue. The type of material and thickness of the sheath may be chosen so that irrigation fluid delivered through the catheter to the expandable assembly can pass outward through the sheath to the tissue, while still preventing blood from penetrating inward through the sheath from the body cavity. The sheath is thus useful in preventing both clotting and tissue damage.

Based on these principles, the disclosed examples provide medical apparatus as described in the appended claims, comprising an insertion tube for insertion into a body cavity of a patient and an expandable assembly connected distally to the insertion tube. A flexible porous sheath is fitted over the expandable assembly so that the sheath contacts the tissue within the body cavity. The expandable assembly comprises electrodes, which apply electrical energy through the sheath to tissue within the body cavity. Although the examples that are described hereinbelow relate specifically to a basket catheter for intracardiac ablation, the principles of the present disclosure may be adapted for use in other sorts of procedures in which electrical energy is applied to biological tissues.

In some examples, an electrical signal generator applies electrical energy to the electrodes on the expandable assembly with an amplitude sufficient to ablate the tissue contacted by the spines. In one example, the electrical signal generator applies bipolar electrical pulses to the electrodes with an amplitude sufficient so that the electrical energy applied from the electrodes through the sheath causes irreversible electroporation (IRE) in the tissue. Additionally or alternatively, the electrical signal generator applies a radio-frequency (RF) current to the electrodes with a power sufficient so that the electrical energy applied from the electrodes through the sheath causes thermal ablation of the tissue.

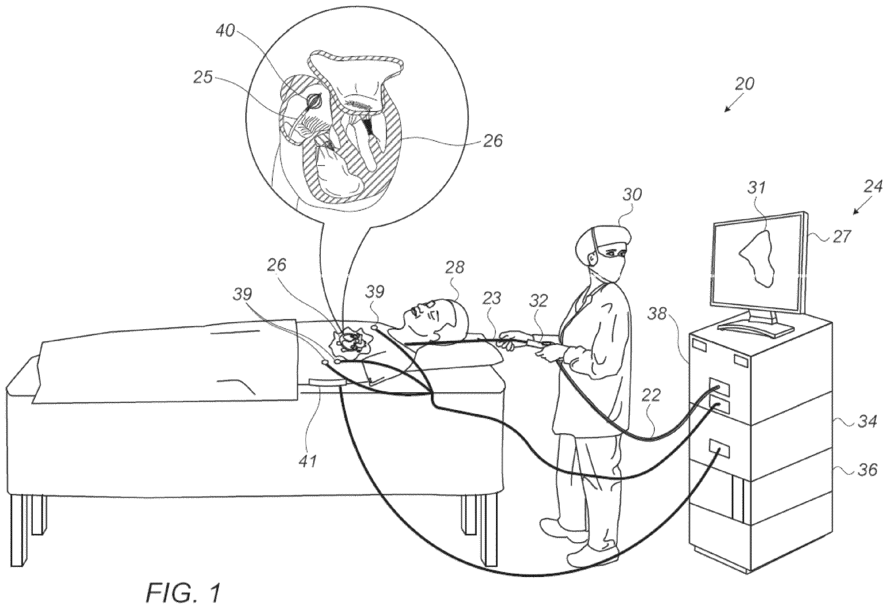

Fig. 1 is a schematic pictorial illustration of a system 20 used in an ablation procedure, in accordance with an example of the disclosure. Elements of system 20 may be based on components of the CARTO^{®} system, produced by Biosense Webster, Inc. (Irvine, California).

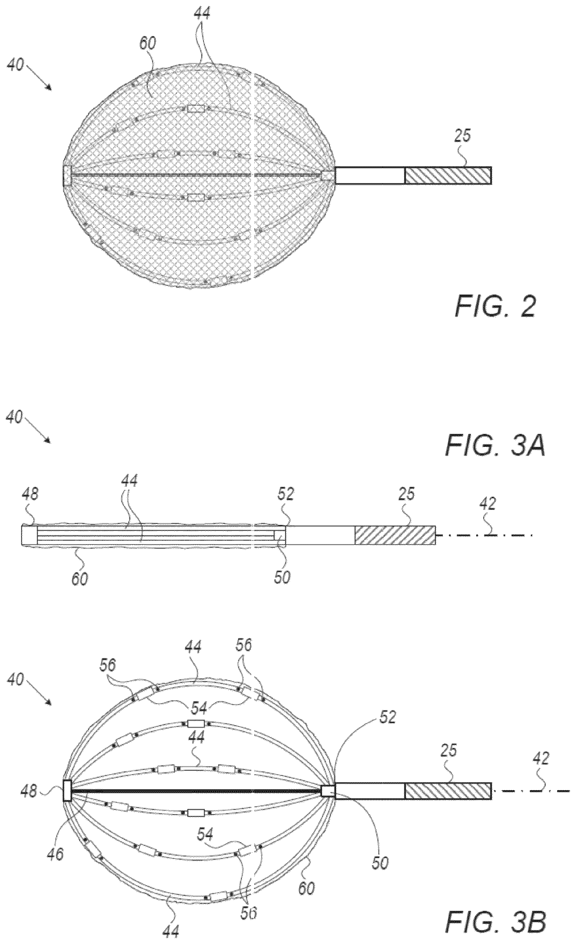

A physician 30 navigates a catheter 22 through the vascular system of a patient 28 into a chamber of a heart 26 of the patient, and then deploys an expandable assembly, such as a basket assembly 40, over which a flexible porous sheath is fitted (as shown in detail in Figs. 2 and 3A/B), at the distal end of the catheter. The proximal end of basket assembly 40 is connected to the distal end of an insertion tube 25, which physician 30 steers using a manipulator 32 near the proximal end of catheter 22. Basket assembly 40 is inserted in a collapsed configuration through a tubular delivery sheath 23, which passes through the vascular system of patient 28 into the heart chamber where the ablation procedure is to be performed. Once inserted into the heart chamber, basket assembly 40 is deployed from the tubular sheath and allowed to expand within the chamber. Catheter 22 is connected at its proximal end to a control console 24. A display 27 on console 24 may present a map 31 or other image of the heart chamber with an icon showing the location of basket assembly 40 in order to assist physician 30 in positioning the basket assembly at the target location for the ablation procedure.

Once basket assembly 40 is properly deployed and positioned in heart 26, physician 30 actuates an electrical signal generator 38 in console 24 to apply electrical energy (such as IRE pulses or RF waveforms) to the electrodes on the basket assembly, under the control of a processor 36. The electrical energy may be applied in a bipolar mode, between pairs of the electrodes on basket assembly 40, or in a unipolar mode, between the electrodes on basket assembly 40 and a separate common electrode, for example a conductive back patch 41, which is applied to the patient's skin. During the ablation procedure, an irrigation pump 34 delivers an irrigation fluid, such as normal saline solution, through insertion tube 25 to basket assembly 40.

Typically, catheter 22 comprises one or more position sensors (not shown in the figures), which output position signals that are indicative of the position (location and orientation) of basket assembly 40. For example, basket assembly 40 may incorporate one or more magnetic sensors, which output electrical signals in response to an applied magnetic field. Processor 36 receives and processes the signals in order to find the location and orientation coordinates of basket assembly 40, using techniques that are known in the art and are implemented, for example, in the above-mentioned Carto system. Alternatively or additionally, system 20 may apply other position-sensing technologies in order to find the coordinates of basket assembly 40. For example, processor 36 may sense the impedances between the electrodes on basket assembly 40 and body-surface electrodes 39, which are applied to the chest of patient 28, and may convert the impedances into location coordinates using techniques that are likewise known in the art. In any case, processor 36 uses the coordinates in displaying the location of basket assembly 40 on map 31.

Alternatively, catheter 22 and the ablation techniques that are described herein may be used without the benefit of position sensing. In such examples, for example, fluoroscopy and/or other imaging techniques may be used to ascertain the location of basket assembly 40 in heart 26.

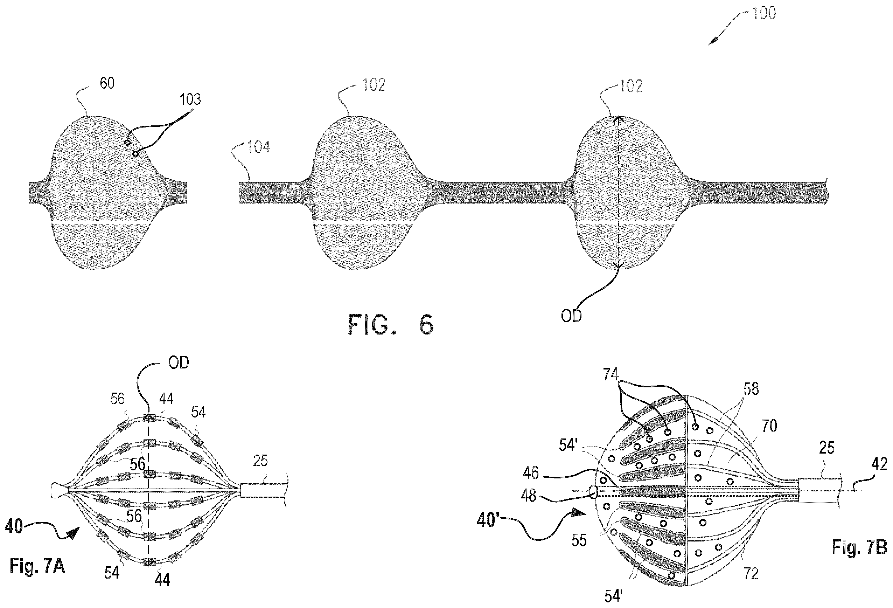

The system configuration that is shown in Fig. 1 is presented by way of example for conceptual clarity in understanding the operation of examples of the present disclosure. For the sake of simplicity, Fig. 1 shows only the elements of system 20 that are specifically related to an expandable assembly, such as basket assembly 40, and ablation procedures using the basket assembly. (Other expandable assemblies to which the principles of the present disclosure may be applied are described hereinbelow with reference to Figs. 7A and 7B.) The remaining elements of the system will be apparent to those skilled in the art, who will likewise understand that the principles of the present disclosure may be implemented in other medical therapeutic systems, using other components. All such alternative implementations are considered to be within the scope of the present disclosure.

Reference is now made to Figs. 2, 3A and 3B, which schematically show details of basket assembly 40, which is covered by a flexible, porous sheath 60 in accordance with an example of the disclosure. Fig. 2 is a side view of basket assembly 40 in its expanded state, while Figs. 3A and 3B are cutaway views showing the basket assembly in collapsed and expanded states, respectively.

Basket assembly 40 has a distal end 48 and a proximal end 50, which is connected to a distal end 52 of insertion tube 25. The basket assembly comprises multiple spines 44, whose proximal ends are conjoined at proximal end 50, and whose distal ends are conjoined at distal end 48. One or more electrodes 54 are disposed externally on each of spines 44. Alternatively, spines 44 may comprise a solid conducting material and may thus serve as electrodes themselves, for example as described in U.S. Patent Application 16/842,648, filed April 7, 2020.

Irrigation outlets 56 in spines 44 allow irrigation fluid flowing within the spines to exit and irrigate tissue in the vicinity of electrodes 54. Alternatively or additionally, the irrigation outlets may be located elsewhere in the basket assembly, for example on an irrigation manifold that is contained inside the basket assembly (not shown in the figures).

Sheath 60 is fitted over basket assembly 40 and thus contacts the tissue in heart 26 when the basket assembly is expanded and advanced against the tissue. Sheath 60 prevents direct contact between spines 44 and the heart tissue. Thus, the electrical energy that is applied to electrodes 54 passes through sheath 60 to the tissue. In one example, sheath 60 comprises expanded polytetrafluoroethylene (ePTFE), for example with a thickness of about 70 µm. The ePTFE sheath is advantageous in being lubricious, smooth, strong, and biocompatible and in preventing spines 44 from becoming embedded in the heart tissue.

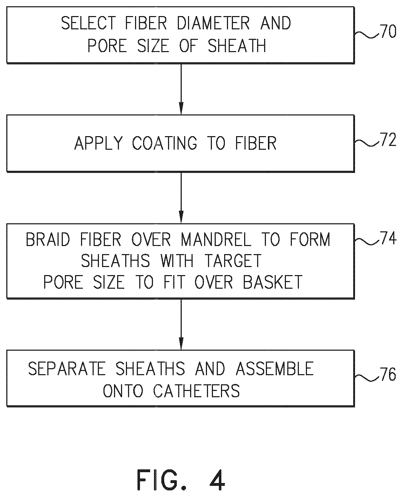

Alternatively, sheath 60 comprises a tube made by braiding suitable polymer fibers, such as a polyethylene terephthalate (PET) or polyamide (nylon) yarn. The tube may be braided with a variable diameter so as to conform better to the deployed basket shape. Specifically, the proximal diameter of the tube may be made to fit the proximal neck of basket, and the distal diameter may be made as small as possible. The distal end may be closed by fastening the loose yarn ends with an adhesive, melting the yarn ends together, or any other suitable method of sealing. An advantage of utilizing a fabric in a tubular shape rather than a flat shape is that the material better conforms to the basket shape, and pleats are avoided or minimized. Avoidance of pleats is helpful in reducing the collapsed diameter of sheath 60 and also reduces the potential for blood to coagulate in the folds of the material. A process for production of this sort of braided sheath is described further hereinbelow with reference to Figs. 4-6.

In yet another example, sheath 60 is made from a sheet of flexible, non-porous material, and pores of the desired size are drilled through the material, for example by laser drilling. In a further example, sheath 60 can be formed by blow-molding a smaller tubular member to form a balloon membrane, with pores subsequently formed through the balloon membrane by laser drilling.

The pores in sheath 60 are sufficiently large to permit the irrigation fluid to pass from irrigation outlets 56 outward through sheath 60 to irrigate the heart tissue, while preventing blood from penetrating inward through the sheath from the heart chamber. The inventors have found it advantageous for this purpose that the pores in the sheath have areas between 10 µm² and 100,000 µm². The best results were obtained with pores having areas between 100 µm² and 10,000 µm². These ranges of pore areas are also useful in ensuring that the electrical energy from electrodes 54 passes freely out through sheath 60 to the adjoining tissue in order to ablate the tissue. Specifically, these ranges of pore areas ensure that irrigation fluid (which is electroconductive) can flow from inside sheath 60 through the pores to the tissue outside the sheath.

The polymer fibers that are used in producing sheath 60, such as PET and nylon fibers, are inherently insulators. Both PET and nylon, however, are hygroscopic, and once the fibers absorb water (or irrigation fluid), they become more conductive and thus enable the electrical energy output by electrodes 54 to pass more freely through sheath 60 to the target tissue. To enhance the performance of sheath 60 in this respect, in one example the polymer fibers are coated with a hydrophilic material. The hydrophilic coating attracts water into the fibers, so that sheath 60 becomes more conductive and thus facilitates efficient ablation. The coating also makes the sheath more lubricious, reducing the force necessary to collapse the basket.

In an alternative example, a hydrophobic coating is applied to the polymer fibers of the sheath. The hydrophobic coating requires the sheath to be pressurized in order for irrigation fluid to flow through it. This positive pressure prevents blood from entering the sheath even when the irrigation is at a low flow rate.#

In the collapsed state of Fig. 3A, spines 44 are straight and aligned parallel to a longitudinal axis 42 of insertion tube 25, to facilitate insertion of basket assembly 40 into heart 26. In this state, sheath 60 collapses inward together with spines 44. To ensure that sheath 60 will collapse with basket assembly 40, sheath 60 is joined to distal end 48 and proximal end 50 of balloon assembly 40. Upon extension of an actuator 46 to separate distal end 48 from proximal end 50, both sheath 60 and spines 44 will compress into a tubular profile of Fig. 3A. Upon retraction of actuator 46 toward proximal end 50, spines 44 and sheath 60 will expand into the spherical like configuration shown in Fig. 3B. In the expanded state of Fig. 3B, spines 44 bow radially outward, causing sheath 60 to expand and contact tissue within the heart.

In one example, spines 44 are produced such that the stable state of basket assembly 40 is the collapsed state of Fig. 3A: In this case, when basket assembly 40 is pushed out of the sheath, it is expanded by drawing actuator 46, such as a suitable wire, in the proximal direction through insertion tube 25. Releasing actuator 46 allows basket assembly 40 to collapse back to its collapsed state.

In another example, spines 44 are produced such that the stable state of basket assembly 40 is the expanded state of Fig. 3B: In this case, basket assembly 40 opens out into the expanded state when it is pushed out of the sheath, and actuator 46 may be replaced by a flexible pusher rod for straightening spines 44 before withdrawing the basket assembly back into the sheath.#

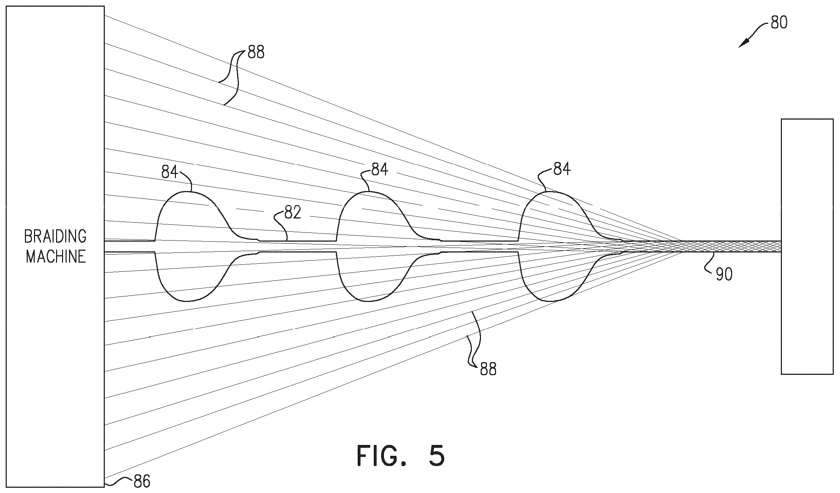

Reference is now made to Figs. 4-6, which schematically illustrate a method for producing sheaths 60 for a catheter basket assembly, in accordance with an example of the disclosure. Fig. 4 is a flow chart showing steps in the method, while Fig. 5 is a schematic side view of a system 80 for producing the sheaths. Fig. 6 is a schematic side view of a braided tube 100 produced using the system of Fig. 5.#

As a preliminary step, the diameter of fibers 88 that are to be used in producing the sheaths and the sizes of the pores to be formed in the sheaths are selected, at a fiber selection step 70. For example, PET or nylon fibers of approximately 25 to 100 denier may be used, and the pores in the sheath may have areas from approximately 10 µm² to approximately 100,000 µm², as noted above. If desired, a hydrophilic or hydrophobic coating may be applied to the fibers, at a coating step 72. #

Fibers 80 are braided over a suitable mandrel 90 to form a tube 100 having a varying diameter, at a braiding step 74. As shown in Fig. 5, mandrel 90 comprises multiple bulbous protrusions 84 disposed along a shaft 82. In one example, bulbous protrusions 84 can comprise a balloon member inflated to a desired shape so that it serves as an underlying support structure for the braiding of the fibers. A braiding machine 86, as is known in the art, braids fibers 88 over mandrel 90. The resulting tube 100, as shown in Fig. 6, comprises bulbs 102 of the desired size, with narrower necks 104 in between. Bulbs 102 are sized to fit over basket assemblies 40, while necks 104 fit snugly over the distal end of insertion tube 25 (as shown in Fig. 2). The braiding parameters of braiding machine 86 are set so that bulbs 102 contain openings (pores 103) of the desired size (for example, of a desired diameter or area) .

To ensure firm contact between sheath 60 and the expandable assembly over which it is to fit, such as basket assembly 40, sheath 60 can be sized to be slightly smaller than the expandable assembly. For example, each bulb 102 (defining the inner diameter of sheath 60) can be sized such that the maximum outer diameter of the bulb 102 is approximately 5% to 20% smaller than the maximum outer diameter (OD) of the expandable assembly over which it is to fit. The maximum OD of the expandable assembly can be measured from the radially outermost points of the expandable assembly (e.g., from one electrode to diametrically opposed electrode or from one spine to a diametrically opposed spine.)

Necks 104 in tube 100 are cut to separate the tube into multiple separate bulbs 102, each of which now becomes a sheath 60, at a sheath separation step 76. Prior to the separation of bulbs 102 into individual sheaths 60, the underlying bulbous protrusions 84 are deflated (if inflated previously) and withdrawn through tube 100. Alternatively, bulbous protrusions 84 may be withdrawn after separation of bulbs 102 into separate pieces. As noted earlier, the distal ends of bulbs 102 are closed after cutting by fastening together the loose ends of fibers 88 with an adhesive, melting the ends together, or any other suitable method of sealing. Sheaths 60 are then fitted over basket assemblies 40.

Fig. 7A and 7B are side views of exemplary expandable members 40 and 40', respectively, which can be used with the sheaths produced from braided tubes as described above. Sheaths 60 (formerly bulbs 102) are fitted over expandable assemblies 40 or 40' by compressing expandable assembly 40 (Fig. 7A) or deflating assembly 40' (Fig. 7B) so that assembly 40 or 40' will fit within the smaller tubular part of sheath 60 (e.g., neck 104).

In the example of Fig. 7B, expandable assembly 40' is in the form of a balloon membrane 70 coupled to a distal end of insertion tube 25. A plurality of electrodes 54' can be disposed on respective substrates 55 disposed on the outer surface of membrane 70. Conductive members 72 can be used to deliver electrical energy to respective electrodes 54'. Conductive members 72 can be disposed inside or outside membrane 70 in the form of electrical traces. Alternatively, conductive members 72 can be wires disposed within the internal volume defined by balloon membrane 70. Conductive members 72 can extend through insertion tube 25 all the way to console 24. Irrigation pores 74 extend through balloon membrane 70 to allow irrigation fluid delivered from insertion tube 25 to flow through membrane 70 and through pores 103 of sheath 60. An actuator 46 (shown by dashed lines) can be mounted inside membrane 70 so that actuator 46 is fixed to distal end 48 and allows for extension of distal end 48 relative to insertion tube 25 (i.e., compressing membrane 70 into a smaller outer profile) or retraction of distal end 48 relative to insertion tube 25 (i.e., causing expansion of membrane 70 into a larger profile). Membrane 70 is typically made of a less flexible material than porous covering 60.

Assembly of expandable member 40' can be completed by deflating membrane 70 and inserting member 40' into the smaller tube (e.g., neck 104) of sheath 60. Thereafter, member 40' can be inflated, and the ends of sheath 60 can be joined to the proximal and distal end of membrane 70. Details of an expandable member 40' of this sort are described in U.S. Patent Application Publication 2021/0169567.

Various features of the disclosure which are, for clarity, described in the contexts of separate examples may also be provided in combination in a single example. Conversely, various features of the disclosure which are, for brevity, described in the context of a single example may also be provided separately or in any suitable subcombination.

It will be appreciated that the examples described above are cited by way of example, and that the present disclosure is not limited to what has been particularly shown and described hereinabove. Rather, the scope of the present disclosure includes both combinations and subcombinations of the various features described hereinabove, as well as variations and modifications thereof which would occur to persons skilled in the art upon reading the foregoing description and which are not disclosed in the prior art. The scope of the invention is defined by the appended claims.

## Claims

1. Medical apparatus (20), comprising:
an insertion tube (25) configured for insertion into a body cavity of a patient (28);
an expandable assembly (40) connected distally to the insertion tube and comprising electrodes (54), which are configured to apply electrical energy to tissue within the body cavity; and
a flexible porous sheath (60), **characterized in that** the flexible porous sheath (60) is fitted over the expandable assembly so that it is covering the expandable assembly and configured to contact the tissue within the body cavity so that the electrical energy is applied from the electrodes through the sheath to the tissue.

2. The apparatus (20) according to claim 1, wherein the sheath (60) comprises a braided polymer fiber.

3. The apparatus (20) according to claim 2, wherein the braided polymer fiber comprises polyethylene terephthalate (PET), or polyamide.

4. The apparatus (20) according to claim 2, wherein the sheath (60) is braided as a tube of varying diameter.

5. The apparatus (20) according to claim 1, wherein the sheath (60) comprises a polymer fiber having a hydrophilic coating or a polymer fiber having a hydrophobic coating.

6. The apparatus (20) according to claim 1, wherein the expandable assembly (40) comprises one or more irrigation outlets (56), which are coupled to convey an irrigation fluid from the insertion tube (25) to the tissue through the sheath.

7. The apparatus (20) according to claim 6, and comprising an irrigation pump (34), which is coupled to supply the irrigation fluid to the insertion tube (25) for conveyance to the irrigation outlets.

8. The apparatus (20) according to claim 1, wherein the expandable assembly (40) comprises a plurality of resilient spines (44), having respective proximal and distal tips, wherein the proximal tips of the spines are joined mechanically at a proximal end (50) of the expandable assembly, and the distal tips of the spines are joined mechanically at a distal end (48) of the expandable assembly, and the spines bow radially outward when the expandable assembly is deployed in the body cavity (28), thereby causing the sheath (60) to contact the tissue in the body cavity.

9. The apparatus (20) according to claim 8, wherein the spines (44) are configured to collapse radially inward so that the spines are aligned along an axis of the insertion tube (25) while the apparatus is being inserted into the body cavity (28).

10. The apparatus (20) according to claim 1, and comprising an electrical signal generator (38) configured to apply electrical energy to the electrodes (54) with an amplitude sufficient to ablate the tissue.

11. The apparatus (20) according to claim 10, wherein the electrical signal generator (38) is configured to apply (i) bipolar electrical pulses to the electrodes (54) with an amplitude sufficient so that the electrical energy applied from the electrodes through the sheath (60) causes irreversible electroporation (IRE) in the tissue, or (ii) a radio-frequency (RF) current to the electrodes with a power sufficient so that the electrical energy applied from the electrodes through the sheath causes thermal ablation of the tissue.

12. The apparatus (20) according to claim 1, wherein the expandable assembly (40') comprises a balloon membrane (70) having an outer surface on which the electrodes (54') are disposed, each of the plurality of electrodes being electrically connected to at least one respective conductive member extending through the insertion tube (25), wherein irrigation pores (74) extend through the balloon membrane to allow irrigation fluid to flow from the insertion tube through the irrigation pores.

13. A method for producing a medical device (20), comprising:
providing an insertion tube (25) configured for insertion into a body cavity of a patient (28);
connecting distally to the insertion tube an expandable assembly (40) comprising electrodes (54); **characterized in that** the method further comprises
fitting a flexible porous sheath (60) over the expandable assembly so that it is covering the expandable assembly, such that the sheath is configured to contact the tissue within the body cavity when the insertion tube is inserted into the body cavity thereby enabling the application of electrical energy from the electrodes through the sheath to the tissue.

14. The apparatus (20) according to claim 1 or the method according to claim 13, wherein the sheath (60) comprises expanded polytetrafluoroethylene (ePTFE).

15. The method according to claim 13, wherein fitting the flexible porous sheath (60) comprises braiding a polymer fiber to form the sheath.

16. The method according to claim 15, wherein the polymer fiber comprises polyethylene terephthalate (PET), or polyamide.

17. The method according to claim 15, wherein braiding the polymer fiber comprises braiding a tube with a varying diameter.

18. The method according to claim 17, wherein braiding the tube comprises braiding polymer fibers over a mandrel comprising multiple bulbous protrusions disposed along a shaft, and cutting the braided tube to form multiple sheaths.

19. The method according to claim 15, and comprising applying a hydrophilic coating to the polymer fiber or applying a hydrophobic coating to the polymer fiber.

20. The apparatus (20) according to claim 6 or the method according to claim 14, wherein the porous sheath (60) contains pores (103) having respective areas between 10 µm² and 100,000 µm², preferably wherein the respective areas of the pores are between 100 µm² and 10,000 µm².

21. The method according to claim 13, wherein the sheath (60) comprises a fabric chosen to permit irrigation fluid to pass outward through the sheath from the one or more irrigation outlets (56) to the tissue while preventing blood from penetrating inward through the sheath from the body cavity (28).

22. The method according to claim 13, wherein connecting the expandable assembly (40) comprises joining together respective distal tips of a plurality of resilient spines (44) at a proximal end (50) of the expandable assembly, and joining together respective distal tips of the spines at a distal end (48) of the expandable assembly, so that the spines are configured to bow radially outward when the expandable assembly is deployed in the body cavity (28), thereby enabling the sheath to contact the tissue in the body cavity.

23. The apparatus (20) according to claim 9 or the method according to claim 22, wherein the spines (44) comprise a conductive material, which is configured to serve as an electrode (54).

24. The method according to claim 22, wherein the spines (44) are configured to collapse radially inward, enabling the spines to be aligned along an axis of the insertion tube (25) while the expandable assembly (40) is being inserted into the body cavity (28).

25. The method according to claim 14, comprising coupling an electrical signal generator (38) configured to apply electrical energy from the electrodes through the sheath to the tissue within the body cavity.

## Patentansprüche

1. Medizinische Einrichtung (20), umfassend:
einen Einführschlauch (25), der für eine Einführung in eine Körperhöhle eines Patienten (28) konfiguriert ist;
eine erweiterbare Anordnung (40), die distal mit dem Einführschlauch verbunden ist und Elektroden (54) umfasst, die konfiguriert sind, um elektrische Energie an Gewebe innerhalb der Körperhöhle anzulegen; und
eine flexible poröse Hülle (60), **dadurch gekennzeichnet, dass** die flexible, poröse Hülle (60) über der erweiterbaren Anordnung eingepasst ist, sodass sie die erweiterbare Anordnung bedeckt und konfiguriert ist, um das Gewebe innerhalb der Körperhöhle zu berühren, sodass die elektrische Energie von den Elektroden durch die Hülle an das Gewebe angelegt wird.

2. Einrichtung (20) nach Anspruch 1, wobei die Hülle (60) eine geflochtene Polymerfaser umfasst.

3. Einrichtung (20) nach Anspruch 2, wobei die geflochtene Polymerfaser Polyethylenterephthalat (PET) oder Polyamid umfasst.

4. Einrichtung (20) nach Anspruch 2, wobei die Hülle (60) als Schlauch mit variierendem Durchmesser geflochten ist.

5. Einrichtung (20) nach Anspruch 1, wobei die Hülle (60) eine Polymerfaser, die eine hydrophile Beschichtung aufweist, oder eine Polymerfaser, die eine hydrophobe Beschichtung aufweist, umfasst.

6. Einrichtung (20) nach Anspruch 1, wobei die erweiterbare Anordnung (40) einen oder mehrere Spülauslässe (56) umfasst, die gekoppelt sind, um ein Spülfluid aus dem Einführschlauch (25) durch die Hülle zu dem Gewebe zu befördern.

7. Einrichtung (20) nach Anspruch 6, und umfassend eine Spülpumpe (34), die gekoppelt ist, um dem Einführschlauch (25) das Spülfluid zur Beförderung an die Spülauslässe zuzuführen.

8. Einrichtung (20) nach Anspruch 1, wobei die erweiterbare Anordnung (40) eine Vielzahl elastischer Dorne (44) umfasst, die jeweilige proximale und distale Spitzen aufweisen, wobei die proximalen Spitzen der Dorne mechanisch an einem proximalen Ende (50) der erweiterbaren Anordnung verbunden sind, und die distalen Spitzen der Dorne mechanisch an einem distalen Ende (48) der erweiterbaren Anordnung verbunden sind und sich die Dorne radial nach außen biegen, wenn die erweiterbare Anordnung in der Körperhöhle (28) angewendet wird, wodurch bewirkt wird, dass die Hülle (60) das Gewebe in der Körperhöhle berührt.

9. Einrichtung (20) nach Anspruch 8, wobei die Dorne (44) konfiguriert sind, um radial nach innen zusammenzufallen, sodass die Dorne entlang einer Achse des Einführschlauchs (25) ausgerichtet sind, während die Einrichtung in die Körperhöhle (28) eingeführt wird.

10. Einrichtung (20) nach Anspruch 1, und umfassend einen elektrischen Signalgenerator (38), der konfiguriert ist, um an die Elektroden (54) elektrische Energie mit einer Amplitude anzulegen, die ausreicht, um das Gewebe abzutragen.

11. Einrichtung (20) nach Anspruch 10, wobei der elektrische Signalgenerator (38) konfiguriert ist, um (i) bipolare elektrische Impulse mit einer ausreichenden Amplitude an die Elektroden (54) anzulegen, sodass die von den Elektroden durch die Hülle (60) angelegte elektrische Energie eine irreversible Elektroporation (IRE) in dem Gewebe verursacht, oder (ii) einen Hochfrequenzstrom (HF-Strom) mit einer ausreichenden Leistung an die Elektroden anzulegen, sodass die von den Elektroden durch die Hülle angelegte elektrische Energie eine thermische Abtragung des Gewebes verursacht.

12. Einrichtung (20) nach Anspruch 1, wobei die erweiterbare Anordnung (40') eine Ballonmembran (70) umfasst, die eine Außenoberfläche aufweist, auf der die Elektroden (54') angeordnet sind, wobei jede der Vielzahl von Elektroden elektrisch mit mindestens einem jeweiligen leitfähigen Element verbunden ist, das sich durch den Einführschlauch (25) erstreckt, wobei sich Spülporen (74) durch die Ballonmembran erstrecken, um zu ermöglichen, dass Spülfluid aus dem Einführschlauch durch die Spülporen fließt.

13. Verfahren zum Herstellen einer medizinischen Vorrichtung (20), umfassend:
Bereitstellen eines Einführschlauchs (25), der für eine Einführung in eine Körperhöhle eines Patienten (28) konfiguriert ist;
Verbinden distal zu dem Einführschlauch einer erweiterbaren Anordnung (40), umfassend Elektroden (54); **dadurch gekennzeichnet, dass** das Verfahren ferner umfasst
Einpassen einer flexiblen, porösen Hülle (60) über die erweiterbare Anordnung, so dass sie die erweiterbare Anordnung auf derartige Weise bedeckt, dass die Hülle konfiguriert ist, um das Gewebe innerhalb der Körperhöhle zu berühren, wenn der Einführschlauch in die Körperhöhle eingeführt wird, wodurch die Anwendung elektrischer Energie von den Elektroden durch die Hülle an das Gewebe ermöglicht wird.

14. Einrichtung (20) nach Anspruch 1 oder Verfahren nach Anspruch 13, wobei die Hülle (60) expandiertes Polytetrafluorethylen (ePTFE) umfasst.

15. Verfahren nach Anspruch 13, wobei das Einpassen der flexiblen porösen Hülle (60) ein Flechten einer Polymerfaser umfasst, um die Hülle auszubilden.

16. Verfahren nach Anspruch 15, wobei die Polymerfaser Polyethylenterephthalat (PET) oder Polyamid umfasst.

17. Verfahren nach Anspruch 15, wobei das Flechten der Polymerfaser ein Flechten eines Schlauchs mit variierendem Durchmesser umfasst.

18. Verfahren nach Anspruch 17, wobei das Flechten des Schlauchs ein Flechten von Polymerfasern über einen Spanndorn umfasst, umfassend mehrere bauchige Vorsprünge, die entlang eines Schafts angeordnet sind, und ein Schneiden des geflochtenen Schlauchs, um mehrere Hüllen auszubilden.

19. Verfahren nach Anspruch 15, und umfassend ein Aufbringen einer hydrophilen Beschichtung auf die Polymerfaser oder ein Aufbringen einer hydrophoben Beschichtung auf die Polymerfaser.

20. Einrichtung (20) nach Anspruch 6 oder Verfahren nach Anspruch 14, wobei die poröse Hülle (60) Poren (103) enthält, die jeweilige Flächen zwischen 10 µm² und 100.000 µm² aufweisen, wobei die jeweiligen Porenflächen vorzugsweise zwischen 100 µm² und 10.000 µm² betragen.

21. Verfahren nach Anspruch 13, wobei die Hülle (60) ein Textil umfasst, das gewählt ist, um Spülfluid von dem einen oder den mehreren Spülauslässen (56) durch die Hülle nach außen zu dem Gewebe durchzulassen, während verhindert wird, dass Blut aus der Körperhöhle (28) durch die Hülle nach innen eindringt.

22. Verfahren nach Anspruch 13, wobei das Verbinden der erweiterbaren Anordnung (40) ein Zusammenfügen der jeweiligen distalen Spitzen einer Vielzahl elastischer Dorne (44) an einem proximalen Ende (50) der erweiterbaren Anordnung und ein Zusammenfügen der jeweiligen distalen Spitzen der Dorne an einem distalen Ende (48) der erweiterbaren Anordnung umfasst, so dass die Dorne konfiguriert sind, um sich radial nach außen zu biegen, wenn die erweiterbare Anordnung in der Körperhöhle (28) angewendet wird, wodurch ermöglicht wird, dass die Hülle das Gewebe in der Körperhöhle berührt.

23. Einrichtung (20) nach Anspruch 9 oder Verfahren nach Anspruch 22, wobei die Dorne (44) ein leitfähiges Material umfassen, das konfiguriert ist, um als eine Elektrode (54) zu dienen.

24. Verfahren nach Anspruch 22, wobei die Dorne (44) konfiguriert sind, um radial nach innen zusammenfallen, wodurch ermöglicht wird, dass die Dorne entlang einer Achse des Einführschlauchs (25) ausgerichtet sind, während die erweiterbare Anordnung (40) in die Körperhöhle (28) eingeführt wird.

25. Verfahren nach Anspruch 14, umfassend ein Koppeln eines elektrischen Signalgenerators (38), der konfiguriert ist, um elektrische Energie von den Elektroden durch die Hülle an das Gewebe innerhalb der Körperhöhle anzulegen.

## Revendications

1. Appareil médical (20), comprenant :
un tube d'insertion (25) conçu pour insertion dans une cavité corporelle d'un patient (28) ;
un ensemble expansible (40) relié distalement au tube d'insertion et comprenant des électrodes (54), qui sont configurées pour appliquer de l'énergie électrique à un tissu au sein de la cavité corporelle ; et
une gaine poreuse flexible (60), **caractérisé en ce que** la gaine poreuse flexible (60) est ajustée par-dessus l'ensemble expansible de sorte qu'elle couvre l'ensemble expansible et est conçue pour venir en contact avec le tissu au sein de la cavité corporelle de sorte que l'énergie électrique est appliquée à partir des électrodes à travers la gaine vers le tissu.

2. Appareil (20) selon la revendication 1, dans lequel la gaine (60) comprend une fibre polymère tressée.

3. Appareil (20) selon la revendication 2, dans lequel la fibre polymère tressée comprend du téréphtalate de polyéthylène (PET) ou du polyamide.

4. Appareil (20) selon la revendication 2, dans lequel la gaine (60) est tressée en tant que tube de diamètre variable.

5. Appareil (20) selon la revendication 1, dans lequel la gaine (60) comprend une fibre polymère ayant un revêtement hydrophile ou une fibre polymère ayant un revêtement hydrophobe.

6. Appareil (20) selon la revendication 1, dans lequel l'ensemble expansible (40) comprend une ou plusieurs sorties d'irrigation (56), qui sont couplées pour transporter un fluide d'irrigation à partir du tube d'insertion (25) vers le tissu, à travers la gaine.

7. Appareil (20) selon la revendication 6, et comprenant une pompe d'irrigation (34), qui est couplée pour alimenter le fluide d'irrigation au tube d'insertion (25) pour un transport vers les sorties d'irrigation.

8. Appareil (20) selon la revendication 1, dans lequel l'ensemble expansible (40) comprend une pluralité d'éléments d'armature élastiques (44), ayant des bouts proximaux et distaux respectifs, dans lequel les bouts proximaux des éléments d'armature sont joints mécaniquement au niveau d'une extrémité proximale (50) de l'ensemble expansible, et les bouts distaux des éléments d'armature sont joints mécaniquement au niveau d'une extrémité distale (48) de l'ensemble expansible, et les éléments d'armature s'incurvent radialement vers l'extérieur lorsque l'ensemble expansible est déployé dans la cavité corporelle (28), amenant de ce fait la gaine (60) à venir en contact avec le tissu dans la cavité corporelle.

9. Appareil (20) selon la revendication 8, dans lequel les éléments d'armature (44) sont conçus pour se replier radialement vers l'intérieur de sorte que les éléments d'armature sont alignés le long d'un axe du tube d'insertion (25) alors que l'appareil est en cours d'insertion dans la cavité corporelle (28).

10. Appareil (20) selon la revendication 1, et comprenant un générateur de signaux électriques (38) configuré pour appliquer de l'énergie électrique aux électrodes (54) avec une amplitude suffisante pour ablater le tissu.

11. Appareil (20) selon la revendication 10, dans lequel le générateur de signaux électriques (38) est configuré pour appliquer (i) des impulsions électriques bipolaires aux électrodes (54) avec une amplitude suffisante de sorte que l'énergie électrique appliquée à partir des électrodes à travers la gaine (60) amène une électroporation irréversible (IRE) dans le tissu, ou (ii) un courant radiofréquence (RF) aux électrodes avec une puissance suffisante de sorte que l'énergie électrique appliquée à partir des électrodes à travers la gaine amène une ablation thermique du tissu.

12. Appareil (20) selon la revendication 1, dans lequel l'ensemble expansible (40') comprend une membrane de ballonnet (70) ayant une surface externe sur laquelle les électrodes (54') sont disposées, chacune parmi la pluralité d'électrodes étant connectée électriquement à au moins un élément conducteur respectif s'étendant à travers le tube d'insertion (25), dans lequel des pores d'irrigation (74) s'étendent à travers la membrane de ballonnet pour permettre à un fluide d'irrigation de s'écouler à partir du tube d'insertion à travers les pores d'irrigation.

13. Procédé permettant de produire un dispositif médical (20), comprenant :
la fourniture d'un tube d'insertion (25) conçu pour insertion dans une cavité corporelle d'un patient (28) ;
le fait de relier distalement au tube d'insertion un ensemble expansible (40) comprenant des électrodes (54) ; **caractérisé en ce que** le procédé comprend en outre
l'ajustement d'une gaine poreuse flexible (60) par-dessus l'ensemble expansible de sorte qu'elle couvre l'ensemble expansible, de telle sorte que la gaine est conçue pour venir en contact avec le tissu au sein de la cavité corporelle lorsque le tube d'insertion est inséré dans la cavité corporelle en permettant de ce fait l'application d'énergie électrique à partir des électrodes à travers la gaine vers le tissu.

14. Appareil (20) selon la revendication 1 ou procédé selon la revendication 13, dans lequel la gaine (60) comprend du polytétrafluoroéthylène expansé (ePTFE).

15. Procédé selon la revendication 13, dans lequel l'ajustement de la gaine poreuse flexible (60) comprend le tressage d'une fibre polymère pour former la gaine.

16. Procédé selon la revendication 15, dans lequel la fibre polymère comprend du téréphtalate de polyéthylène (PET) ou du polyamide.

17. Procédé selon la revendication 15, dans lequel le tressage de la fibre polymère comprend le tressage d'un tube avec un diamètre variable.

18. Procédé selon la revendication 17, dans lequel le tressage du tube comprend le tressage de fibres polymères par-dessus un mandrin comprenant de multiples parties saillantes en forme de bulbe disposées le long d'une tige, et la découpe du tube tressé pour former de multiples gaines.

19. Procédé selon la revendication 15, et comprenant l'application d'un revêtement hydrophile à la fibre polymère ou l'application d'un revêtement hydrophobe à la fibre polymère.

20. Appareil (20) selon la revendication 6 ou procédé selon la revendication 14, dans lequel la gaine poreuse (60) contient des pores (103) ayant des aires respectives entre 10 µm² et 100 000 µm², de préférence dans lequel les aires respectives des pores sont comprises entre 100 µm² et 10 000 µm².

21. Procédé selon la revendication 13, dans lequel la gaine (60) comprend un tissu choisi pour permettre à un fluide d'irrigation de passer à l'extérieur à travers la gaine à partir de la ou des sorties d'irrigation (56) vers le tissu tout en empêchant du sang de pénétrer à l'intérieur à travers la gaine à partir de la cavité corporelle (28).

22. Procédé selon la revendication 13, dans lequel le fait de relier l'ensemble expansible (40) comprend le fait de réunir des bouts distaux d'une pluralité d'éléments d'armature élastiques (44) au niveau d'une extrémité proximale (50) de l'ensemble expansible, et le fait de réunir des bouts distaux respectifs des éléments d'armature au niveau d'une extrémité distale (48) de l'ensemble expansible, de sorte que les éléments d'armature sont conçus pour s'incurver radialement vers l'extérieur lorsque l'ensemble expansible est déployé dans la cavité corporelle (28), en permettant de ce fait à la gaine de venir en contact avec le tissu dans la cavité corporelle.

23. Appareil (20) selon la revendication 9 ou procédé selon la revendication 22, dans lequel les éléments d'armature (44) comprennent un matériau conducteur, qui est conçu pour servir d'électrode (54).

24. Procédé selon la revendication 22, dans lequel les éléments d'armature (44) sont conçus pour se replier radialement vers l'intérieur, en permettant aux éléments d'armature d'être alignés le long d'un axe du tube d'insertion (25) alors que l'ensemble expansible (40) est en cours d'insertion dans la cavité corporelle (28).

25. Procédé selon la revendication 14, comprenant le couplage d'un générateur de signaux électriques (38) configuré pour appliquer de l'énergie électrique à partir des électrodes à travers la gaine vers le tissu au sein de la cavité corporelle.
